# EUROPEAN PATENT SPECIFICATION

(11) **EP 3 936 107 B1**
(45) Date of publication and mention of the grant of the patent: **21.02.2024**
(21) Application number: 20766762.7
(22) Date of filing: 03.03.2020
(51) Int. Cl.: A61J 1/20, A61J 1/14, A61M 5/19, A61M 5/178, A61M 5/31

(54) **DRUG MIXING KIT SYRINGE AND DRUG MIXING KIT**
ARZNEIMITTELMISCHKITNADEL UND ARZNEIMITTELMISCHKIT
SERINGUE DE KIT DE MÉLANGE DE MÉDICAMENT ET KIT DE MÉLANGE DE MÉDICAMENT

(30) Priority: 05.03.2019 KR 20190025414
(43) Date of publication of application: 12.01.2022
(73) Proprietor: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2020/002988
(87) International publication number: WO 2020/180081

(56) References cited:
- JP-A- H10 165 502
- KR-A- 20150 116 860
- US-A1- 2002 087 118
- US-A1- 2002 177 819
- US-A1- 2007 088 252
- US-B2- 7 731 679

## Description

### TECHNICAL FIELD

The present disclosure relates to a syringe, a drug mixing device, a drug mixing kit, and a method of manufacturing the same.

### BACKGROUND

In the medical field, a mixed drug in a liquid form may be injected into a patient by mixing a liquid or powder form, such as an analgesic, antibiotic, or anticancer drug, with another drug in a liquid form, a medical liquid, or a distilled water. An example of a method of mixing drugs in the related art is as follows. A container, such as a bag or vial containing a drug in a liquid form, a medical liquid, or a distilled water, is punctured by piercing the container with an injection needle of a syringe, the content inside the container is sucked into and accommodated in the syringe, and the injection needle is withdrawn from the container. Then, another container containing a drug in a liquid or powder form is punctured by piercing the other container with the injection needle of the syringe, the drug in the liquid form, the medical liquid or the distilled water inside the syringe is injected into the other container, and then the other container is shaken to mix the contents therein. After the mixing is completed, the mixed solution in the other container is sucked into the syringe through the injection needle to be accommodated in the syringe, and then the injection needle is withdrawn from the other container.

### SUMMARY

In the related art, in order to mix the contents in two containers with each other, there is a problem in that it is necessary to sequentially pierce the two containers with an injection needle of a syringe and to withdraw the injection needle of the syringe. Due to complicated manual work in an urgent medical field, there is a problem in that the probability that the injection needle of the syringe is contaminated by external contaminants or pathogens increases and that the probability of an accident in which the injection needle touches the skin of medical staff increases. This is a very serious problem as it may have a significant adverse effect on the health of a patient and medical staff. Embodiments of the present disclosure solve the problems of the related art described above.

In the case of using an expensive sterilization facility in order to prevent the above-described problems in the related art, there is a problem that it is difficult to use such a sterilization facility in an emergency situation in the medical field while incurring a high cost. Embodiments of the present disclosure solve the problems described above.

US 7 731 679 discloses a drug mixing kit as defined in the preamble of claim 1. US 2002/177819 discloses a device for injecting medicine from a pre-filled vial into a patient through an injection needle.. The device operates by pumping medicine out of the vial into a transfer chamber. Preferably, the device includes an injector assembly and a vial holder assembly. The vial holder assembly is attachable with the injector assembly and is operable to transfer the medicine out of the vial and into the transfer chamber to prepare the device for an injection.

An aspect of the present disclosure provides embodiments of a drug mixing kit for mixing a first content in a liquid form in a first container with a second content in a liquid or powder form in a second container, as defined in claim 1. The invention is defined by the appended claims.

In the embodiments, the syringe may further include a rod configured to pressurize the first container by being inserted into and moved in the introduction space.

In the embodiments, the plunger may be configured to open the introduction space in one direction. The protruding direction of the inner needle may be the one direction.

In the embodiments, the plunger may be configured to be movable with respect to the syringe housing in the one direction.

In the embodiments, an outer flow path configured to guide the flow of the liquid between the accommodation space and an exterior of the syringe is formed in the syringe.

In the embodiments, the syringe may further include a syringe needle forming therein at least a portion of the outer flow path and protruding to an exterior of the syringe housing.

In the embodiments, the container connection part may include a plurality of hooks configured to be engaged with the second container. The body may include a support part configured to support the container connection part.

In the embodiments, the drug mixing device may further include an outer needle forming a side of the other end of the drug flow path and protruding to be insertable into an interior of the second container. The plurality of hooks of the container connection part may be arranged to be spaced apart from each other in a circumferential direction around the outer needle.

In the embodiments, the drug mixing device may further include an outer needle forming a side of the other end of the drug flow path and protruding to be insertable into an interior of the second container.

In the embodiments, the drug mixing device may further include a supporter coupled to the body and configured to support the syringe.

Another aspect of the present disclosure provides embodiments of a method for manufacturing a drug mixing kit. The method of manufacturing the drug mixing kit according to a representative embodiment includes fixing the container connection part to the support part.

According to the embodiments of the present disclosure, it is possible to significantly reduce the probability that a component inserted into a patient, such as an injection needle, or a drug administered to the patient is contaminated from external contamination sources or pathogens.

According to the embodiments of the present disclosure, it is possible for medical staff to mix drugs in a very simple and efficient manner.

According to the embodiments of the present disclosure, it is possible to quickly and safely mix drugs.

### BRIEF DESCRIPTION OF THE DRAWINGS

FIG. 1 is a perspective view illustrating the state in which a first container 6 and a second container 7 are coupled to a drug mixing kit 1 according to a first embodiment of the present disclosure.
FIGS. 2A to 2F are vertical cross-sectional views illustrating a drug mixing device 100, a syringe 200, the first container 6, and/or the second container 7 taken along line S1-S1' in FIG. 1 and sequentially showing an example of using the drug mixing kit 1 of FIG. 1.
FIG. 3 is a perspective view illustrating the state in which a first container 6 and a second container 7 are coupled to a drug mixing kit 1' according to a second embodiment of the present disclosure.
FIG. 4 is a vertical cross-sectional view illustrating the drug mixing kit 1' of FIG. 3.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in this disclosure, expressions such as "comprising", "including", "having", and the like are to be understood as open-ended terms having the possibility of encompassing other embodiments, unless otherwise mentioned in the phrase or sentence containing such expressions.

The singular form used in the present disclosure may include a plural meaning unless otherwise mentioned. This applies to the singular form recited in the claims.

Terms such as "first" and "second" used in the present disclosure are used in order to distinguish a plurality of components from one another, and do not limit the order or importance of the corresponding components.

In the present disclosure, where it is mentioned in the present disclosure that one element is "connected" to another element, it is to be understood that the one element may be directly connected to the another element, or may be connected to the another element via a new additional element.

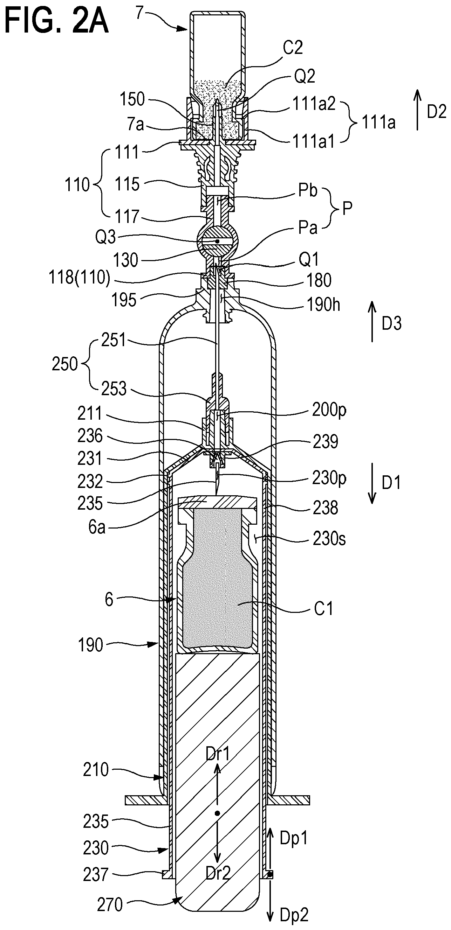

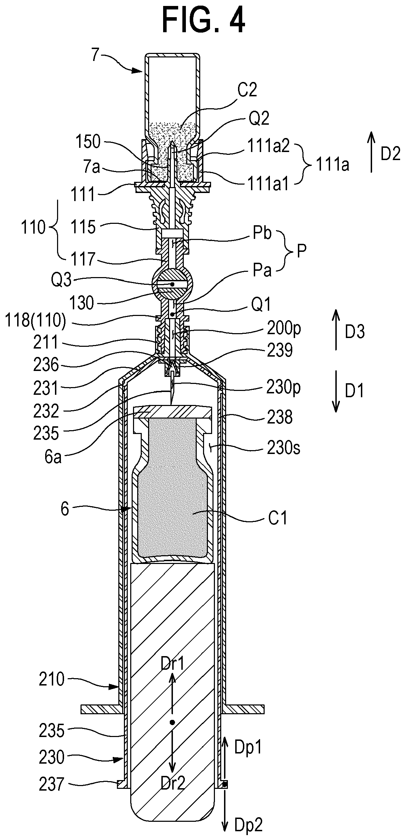

As used in the present disclosure, the term "first direction" means a direction in which a syringe moves when the syringe is brought close to a first container such that the syringe and the first container are coupled to each other, and the term "second direction" means a direction in which a drug mixing device moves when the drug mixing device is brought close to a second container such that the drug mixing device and the second container are coupled to each other. As used in the present disclosure, the "third direction" means a direction in which the syringe moves when the syringe is brought close to the drug mixing device such that the drug mixing device and the syringe are coupled to each other. FIGS. 2A and 4 illustrate the first direction D1, the second direction D2, and the third direction D3, but each direction is not limited to the embodiments of the present disclosure. In the present embodiment, the second direction D2 and the third direction D3 are illustrated as being the same direction, but of course, the second direction and the third direction may be implemented differently.

Hereinafter, embodiments of the present disclosure will be described with reference to the accompanying drawings. In the accompanying drawings, like or relevant components are indicated by like reference numerals. In the following description of embodiments, repeated descriptions of the identical or relevant components will be omitted. However, even if a description of a component is omitted, such a component is not intended to be excluded in an embodiment.

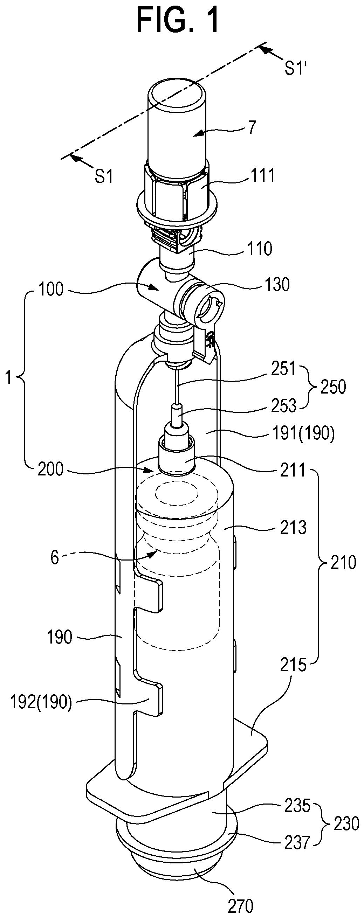

FIG. 1 is a perspective view illustrating a state in which a first container 6 and a second container 7 are coupled to a drug mixing kit 1 according to a first embodiment of the present disclosure. FIG. 2A is a vertical cross-sectional view illustrating the drug mixing kit 1 of FIG. 1, taken along line S1-S1'.

Referring to FIGS. 1 and 2A, a user (e.g., medical staff such as a nurse or a doctor) may couple the first container 6 and the second container 7 to the drug mixing kit 1. The drug mixing kit 1 is configured to mix a first content in a liquid form in the first container 6 with a second content in a liquid or powder form in the second container 7.

The user may mix the first content with the second content using the drug mixing kit 1, and cause the mixture in which the first content and the second content are mixed to flow into a syringe 200. For example, the first content, such as a medical liquid, and the second content, which is a drug in a powder form, may be mixed. As another example, the first content, which is a drug in a liquid form, and the second content, which is another drug in a liquid form, may be mixed.

The drug mixing kit 1 includes the syringe 200 and a drug mixing device 100 coupled to the syringe 200. The drug mixing device 100 and the syringe 200 may be detachably coupled to each other. The first container 6 may be coupled to the syringe 200, and the second container 7 may be coupled to the drug mixing device 100. The user may mix the first content with the second content using the drug mixing kit 1, and may separate the syringe 200 from the drug mixing device 100 after the mixture is filled in the syringe 200.

The drug mixing device 100 includes a drug flow path P configured to guide a flow of a liquid. The drug flow path P includes a first flow path Pa and a second flow path Pb. In the present embodiment, the drug flow path P may extend in a straight line, but according to an embodiment, the drug flow path P may be bent or curved in some sections.

In the drug mixing device 100, the drug flow path P extends from one end Q1 configured to allow the liquid in the accommodation space 200s in the syringe 200 to flow into the drug flow path P therethrough to other end Q2 configured to allow the liquid in the second container 7 to flow into the drug flow path P therethrough. The drug flow path P is located in the drug mixing device 100. For example, the first content in a liquid form in the accommodation space 200s may flow into the drug flow path P, and the mixture in a liquid form in the second container 7 may flow into the drug flow path P.

The one end Q1 of the drug flow path P is disposed on the syringe 200 side. The other end Q2 of the drug flow path P is disposed on the second container 7 side. The one end Q1 is located at the distal end in the first direction D3 of the drug flow path P. The other end Q2 is located at the distal end in the second direction D2 of the drug flow path P.

The drug flow path P includes a first flow path Pa and a second flow path Pb. The first flow path Pa and the second flow path Pb are divided with reference to a point Q3 at which a valve 130 is disposed in the drug flow path P. The first flow path Pa may extend while connecting the one end Q1 and the point Q3 to each other. The second flow path Pb may extend while connecting the other end Q2 and the point Q3 to each other. The first flow path Pa includes a portion extending in the third direction D3. The second flow path Pb includes a portion extending in the second direction D2.

In the embodiment of the drug mixing device 100 separated from the syringe 200, the first flow path Pa may be blocked by a sealer 180. In the embodiment of the drug mixing kit 1 in which the syringe 200 and the drug mixing device 100 are coupled to each other, the first flow path Pa may be opened by a syringe needle 250 penetrating the sealer 180.

At least a portion of the syringe needle 250 of the syringe 200 may be inserted into the first path Pa of the drug mixing device 100. In this case, the liquid may flow along the flow path inside the syringe needle 250 disposed in the first flow path Pa. The syringe needle 250 is inserted into the first flow path Pa in the third direction D3.

The drug mixing device 100 includes a body 110. At least a portion of the drug flow path P may be formed in the body 110. The drug flow path P may penetrate the body 110. A portion of the first flow path Pa and a portion of the second flow path Pb may be formed in the body 110.

The body 110 may be configured to be coupled with the second container 7. The body 110 includes a container connection part 111 configured to be couplable with the second container 7. The body 110 includes a syringe connection part 118 configured to be connectable with the syringe. The body 110 includes support parts 115 and 117 configured to support the container connection part 111. The support parts 115 and 117 may support the syringe connection part 118.

The support parts 115 and 117 may include a first part 115 and a second part 117 coupled to each other. The first part 115 and the second part 117 may be coupled to each other through a method such as laser bonding or ultrasonic bonding. The drug flow path P may penetrate the first part 115 and the second part 117.

The first part 115 may support the container connection part 111. In the present embodiment, the container connection part 111 is attached to the support parts 115 and 117. In another embodiment (not illustrated), the first part 115 and the container connection part 111 may be integrally formed through a method such as injection molding or the like.

An outer needle 150 may be fixed to the first part 115. In the present embodiment, the outer needle 150 is integrally molded with the part 115 of the body 110. In another embodiment (not illustrated), the outer needle 150 may be manufactured as a member separate from the body 110 and fixed to the body 110.

The second part 117 may support the syringe connection part 118. In the present embodiment, the syringe connection part 118 is molded integrally with the second part 117. In another embodiment (not illustrated), the syringe connection part 118 may be manufactured as a member separate from the support parts 115 and 117 and fixed to the support parts 115 and 117.

Avalve 130 may be disposed in the second part 117. A portion of the valve 130 may be exposed to an exterior of the second part 117 such that the user is able to operate the exposed portion of the valve 130 to open/close the drug flow path P.

In the present embodiment, the container connection part 111 is configured to be hooked with the second container 7, but in another embodiment (not illustrated), the container connection part may be configured to be coupled with the corresponding second container through another method such as screwing.

In an embodiment, the container connection part 111 includes a plurality of hooks 111a configured to be engaged with the second container 7. In the state in which the second container 7 is coupled to the drug mixing device 100, the plurality of hooks 111a may be disposed to be spaced apart from each other in the circumferential direction around the position of the second container 7. The plurality of hooks 111a may be arranged to be spaced apart from each other in the circumferential direction around the outer needle 150. Each hook 111a includes a hook extension 111a1 extending in the second direction D2 from the support parts 115 and 117, and an engagement protrusion 111a2 protruding from the distal end in the second direction D2 of the hook extension 111a1 toward the center (in the direction facing the outer needle).

The syringe connection part 118 forms therein at least a portion of the first flow path Pa configured to be capable of discharging the liquid into the syringe 200. In the first embodiment, in the state in which the sealer 180 is penetrated by the syringe needle 250, the liquid may flow through the first flow path Pa.

The syringe connection part 118 may protrude from the support parts 115 and 117 in a direction opposite to the third direction D3. The distal end of the first flow path Pa is disposed on a side surface of the syringe connection part 118 in a direction opposite to the third direction D3. In the first embodiment, the syringe connection part 118 is connected to a supporter 190. In the present embodiment, the syringe connection part 118 and the supporter 190 are coupled to each other through laser or ultrasonic bonding, but in another embodiment (not illustrated), the syringe connection part 118 and the supporter 190 may be coupled to each other through a fastening method such as screwing.

The syringe connection part 118 is configured to be connectable with the syringe 200, which forms therein the accommodation space 200s in which a liquid is accommodated. In the present embodiment, the syringe connection part 118 is connected to the syringe 200 via a sealer 180. In the state in which the drug mixing device 100 and the syringe 200 are coupled to each other, the accommodation space 200s communicates with the first flow path Pa.

The drug mixing device 100 includes a valve 130 disposed in the drug flow path P. The valve 130 is configured to open/close a flow of the liquid in the drug flow path P. For example, the valve 130 may be configured to control the opening/closing of the drug flow path P through rotation.

The drug mixing device 100 includes an outer needle 150 protruding to be insertable into an interior of the second container 7. The outer needle 150 may be formed in the form of an injection needle. The outer needle 150 protrudes in the second direction D2. At the inlet of the second container 7, a film 7a made of a rubber material may be formed, and when the user couples the second container 7 to the drug mixing device 100, the outer needle 150 may penetrate the film 7a.

The outer needle 150 is fixed to the body 110. The outer needle 150 may be fixed to the first part 115. In another embodiment (not illustrated), the outer needle 150 may be fixedly inserted into the body 110. The outer needle 150 forms therein at least a portion of the drug flow path P. The outer needle 150 may form therein a portion of the drug flow path P extending from the body 110 in the second direction D2. The outer needle 150 forms a side of the other end Q2 of the drug flow path P.

The outer needle 150 includes a pointed end formed in the first direction D1 and a support end formed on the opposite side to the pointed end. A pointed end hole (not illustrated) is formed at the pointed end, and a support end hole (not illustrated) is formed at the support end. The outer needle 150 forms therein a portion of the drug flow path P interconnecting the pointed end hole and the support end hole.

The drug mixing device 100 may include a sealer 180 disposed in a partial flow path Pa of the drug flow path P extending to a side of the one end Q1 with reference to the point Q3 at which the valve 130 is disposed in the drug flow path P. In the present embodiment, the sealer 180 is disposed at the distal end of the first flow path Pa. The sealer 180 is configured to be penetrated by the syringe needle 250 protruding to an exterior of the syringe housing 210. The sealer 180 may be formed of a silicon material. The user may separate the syringe 200 from the drug mixing device 100 by withdrawing the syringe needle 250 from the sealer 180.

The drug mixing device 100 may include a supporter 190 configured to support the syringe 200. The supporter 190 is coupled to the body. The supporter 190 may be coupled to the syringe connection part 118. The supporter 190 includes a body coupling portion 195 coupled to the body 110. A connection hole 190h through which the syringe needle 250 passes is formed in the center of the body coupling portion 195. The connection hole 190h may penetrate the supporter 190 in the third direction D3. The connection hole 190h may face the sealer 180 in the third direction.

The supporter 190 includes a support surface 191 that forms a surface to come into contact with the outer surface of the syringe 200. For example, the support surface 191 may form an inner peripheral surface corresponding to the outer peripheral surface of the syringe. The support surface 191 may form an arc on a cross-section perpendicular to the third direction D3. The support surface 191 extends in the third direction D3.

The supporter 190 includes a guide protrusion 192 protruding from the support surface 191 in the extension direction of the support surface 191. The guide protrusion 192 may guide the coupling and/or separation of the syringe 200 and the drug mixing device 100.

The syringe 200 forms therein an accommodation space 200s communicating with the first flow path Pa. The syringe 200 is configured to be capable of sucking the liquid from the first flow path Pa into the accommodation space 200s. The syringe 200 is configured to be capable of discharging the liquid from the accommodation space 200s to the first flow path Pa. The accommodation space 200s is a space capable of accommodating the liquid. The volume of the accommodation space 200s is changed by the relative motion of the plunger 230 with respect to the syringe housing 210, and thus the liquid is sucked into or discharged from the accommodation space 200s.

The syringe 200 includes an outer flow path 200p configured to guide a flow of the liquid between the accommodation space 200s and an exterior of the syringe 200. In the state in which the drug mixing device 100 and the syringe 200 are coupled to each other, the outer flow path 200p guides a flow of the liquid between the accommodation space 200s and the chemical solution flow path P. The outer flow path 200p interconnects the first flow path Pa and the accommodation space 200s. The syringe needle 250 may form therein at least a portion of the outer flow path 200p. The syringe housing 210 may form therein at least a portion of the outer flow path 200p. In the state in which the syringe 200 and the drug mixing device 100 are coupled to each other, a portion of the outer flow path 200p may be disposed in the first flow path Pa.

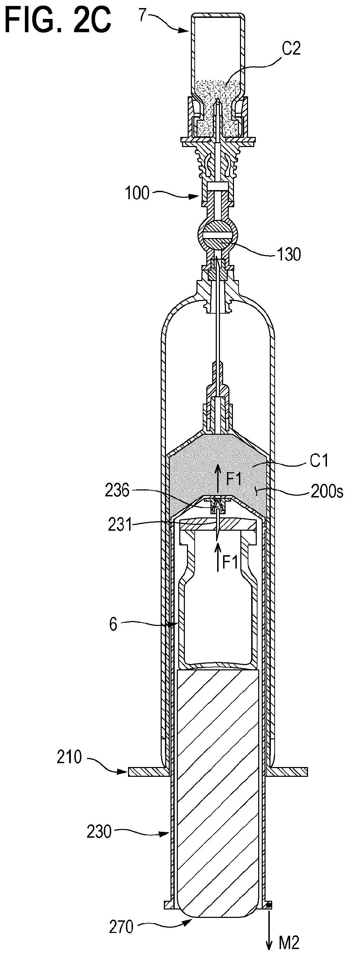

The syringe 200 includes a syringe housing 210 that forms the exterior thereof. The syringe housing 210 forms a space therein. The accommodation space 200s may be disposed in the syringe housing 210 *(see* FIG. 2C). A syringe hole (not illustrated) communicating with the accommodation space 200s is formed in the syringe housing 210. The syringe hole may form a portion of the outer flow path 200p. The syringe housing 210 includes a coupling portion 211 coupled to the syringe needle 250. The syringe hole may be disposed in the center of the coupling part 211. The syringe housing 210 includes a syringe body 213 extending in a cylindrical shape. The syringe housing 210 may include an engagement portion 215 protruding outward from the syringe body part 213 perpendicular to the third direction D3 to be engaged with a user's hand.

The syringe 200 includes a plunger 230 configured to be movable inside the syringe housing 210. The plunger 230 is configured to change the volume of the accommodation space 200s, which accommodates the syringe housing 210 therein, by being inserted into and moved in the syringe housing 210. The accommodation space 200s is partitioned by a pressing surface 231 of the plunger 230 and the inner surface of the syringe housing 210. The plunger 230 is configured to be movable in a discharge motion direction Dp1 in which the pressing surface 231 faces the accommodation space 200s and the inflow motion direction Dp2 opposite to the discharge motion direction Dp1.

An introduction space 230s into which the first container 6 is introduced is formed inside the plunger 230. The plunger 230 may be configured such that the introduction space 230s is opened in one direction Dp2. The plunger 230 may be configured to be movable in the one direction Dp2 with respect to the syringe housing 210. The plunger 230 may be configured to be movable in the one direction Dp2 and the opposite direction Dp1 to the one direction.

The plunger 230 includes a gasket 232 that is in contact with the inner surface of the syringe housing. The plunger 230 includes a plunger housing 238 to which the gasket 232 is fixed. The introduction space 230s may be formed inside the plunger housing 238. The plunger housing 238 extends to an exterior of the opening of one side of the syringe housing 210. A manipulation portion 237 configured to be brought into contact and engaged with the user's hand is provided at the distal end of the plunger housing 238 in the inflow motion direction Dp2.

The plunger 230 may include an inner flow path 230p that allows the introduction space 230s and the accommodation space 200s to communicate with each other therethrough. The inner flow path 230p guides a flow of the liquid from the introduction space 230s to the accommodation space 200s. The inner needle 235 may form therein at least a portion of the inner flow path 230p. The plunger housing 238 may form therein at least a portion of the inner flow path 230p.

The plunger 230 includes an inner needle 235 protruding toward an interior of the introduction space 230s. The protruding direction of the inner needle 235 may be the one direction Dp2. This makes it possible for the inner needle 235 to penetrate the second container 7 while the second container 7 is inserted into the syringe.

The plunger 230 includes the inner needle 235 protruding to be insertable into an interior of the first container 6. The inner needle 235 may be formed in the form of an injection needle. The inner needle 235 protrudes in the first direction D1. Here, the first direction D1 and the inflow motion direction Dp2 may be the same as each other. At the inlet of the first container 6, a film 6a made of a rubber material may be formed, and when the user couples the first container 6 to the drug mixing device 100, the inner needle 235 may penetrate the film 6a.

The inner needle 235 may be fixed to the plunger housing 238. The inner needle 235 may form therein a portion of the inner flow path 230p extending from the plunger housing 238 in the first direction D1.

The inner needle 235 includes a pointed end formed in the first direction D1 and a support end formed on the opposite side to the pointed end. A pointed end hole (not illustrated) is formed at the pointed end of the inner needle 235, and a support end hole (not illustrated) is formed at the support end. The inner needle 235 forms therein a portion of the inner flow path 230p interconnecting the pointed end hole and the support end hole.

The plunger 230 includes a backflow prevention part 236 disposed in the inner flow path 230p. The backflow prevention part 236 is configured to substantially prevent a flow of the liquid from a side of the accommodation space 200s to a side of the introduction space 230s. The backflow prevention part 236 implements the function of a check valve (a one-way valve). The backflow prevention part 236 may allow a flow of the liquid moving from the introduction space 230s to the accommodation space 200s (inflow flow), but may block a flow of the liquid moving from the accommodation space 200s to the introduction space 230s (outflow flow). Various types of backflow prevention parts capable of implementing the function of a check valve may be configured.

In the present embodiment, the backflow prevention part 236 includes a protrusion (not illustrated) protruding in the direction of the inflow flow. In the present embodiment, the protrusion of the backflow prevention part 236 protrudes in an opposite direction to the first direction D1. The protrusion is formed of a flexible material.

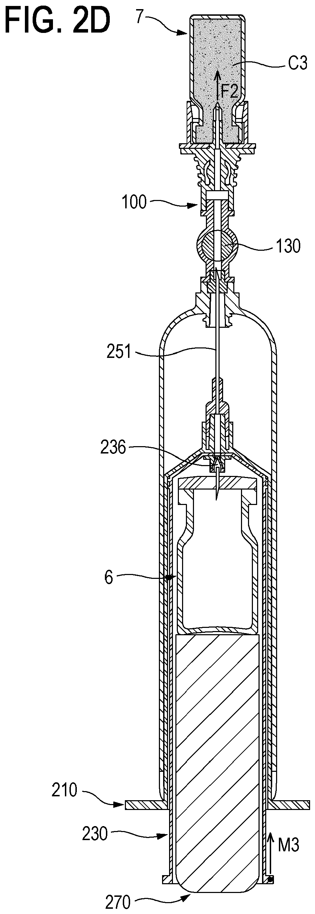

A hole (not illustrated) is formed at the protruding distal end of the protrusion of the backflow prevention part 236. The hole in the protrusion is formed to allow the liquid to pass therethrough. The hole of the protrusion is opened or closed depending on the flow direction of the liquid in the inner flow path 230p. When the liquid in the inner flow path 230p flows in the direction F1 of the inflow flow, the hole in the protrusion is opened (*see* FIG. 2C). The hole in the protrusion is closed when there is no flow of the liquid in the inner flow path 230p or when the liquid is about to flow in the outflow direction (*see* FIG. 2D).

The backflow prevention part 236 includes a seating portion (not illustrated) seated on a fixing part 239. The seating portion supports the protrusion. A hole may be formed in the center of the seating portion, and the liquid may move through the hole in the seating portion to the hole in the protrusion.

For example, the protrusion of the backflow prevention part 236 may be formed in a conical shape as a whole such that the vertex portion thereof protrudes. In this case, the hole in the protrusion is formed in the vertex portion.

As another example, the protrusion may include a first oblique surface extending to be inclined with respect to the protruding direction, a second oblique surface extending to be inclined in a direction opposite to the first oblique surface with respect to the protruding direction, and both side surfaces that cover the both sides of the first oblique surface and the second oblique surface. In this case, the first oblique surface and the second oblique surface meet while forming an edge at the protruding distal end of the protrusion, and the hole in the protrusion extends along the edge. When the liquid in the inner flow path 230p flows in the inflow direction, the protruding distal end of the first oblique surface and the protruding distal end of the second oblique surface are bent in opposite directions to open the hole in the protrusion.

The plunger 230 may include a fixing part 239 on which the backflow prevention part 236 is seated. The fixing part 239 may be fixed to the plunger housing 238. The fixing part 239 may form therein at least a portion of the inner flow path 230p. A groove recessed in the protruding direction of the protrusion of the backflow prevention part 236 may be formed in the fixing part 239, and the protrusion of the backflow prevention part 236 may be inserted into the groove of the fixing part 239.

The syringe 200 includes a syringe needle 250 protruding to the exterior of the syringe housing 210. The syringe needle 250 is configured to penetrate the sealer 180. The syringe needle 250 forms therein a flow path configured to guide a flow of the liquid between the drug flow path P and the accommodation space 200s.

The syringe needle 250 may protrude in the third direction D3 to form a pointed end. The syringe needle 250 includes a needle spike 251 configured to be insertable into a skin of a patient and a needle support 253 configured to support the needle spike 251. The needle support 253 is fixed to the syringe housing 210.

The syringe 200 may include a rod 270 configured to be capable of pressurizing the first container 6 by being inserted into and moving in the introduction space 230s. The rod 270 may be configured to be movable in the first direction D1 and the opposite direction to the first direction D1. When the rod 270 moves in a pressurizing direction Dr1 that is opposite to the first direction D1, the rod 270 pressurizes the first container 6 so that the inner needle 235 can be inserted into the first container 6. As the rod 270 moves in a withdrawal direction Dr2 which is the first direction D1, the rod 270 may be separated from the plunger 230.

Meanwhile, a method for manufacturing the drug mixing device 100 (manufacturing method) may include a step of manufacturing the container connection part 111 of the body 110. The container connection part 111 may be manufactured according to a design content that meets product specifications. The container connection part 111 may be injection-molded.

The manufacturing method may include a step of fixing the container connection part 111 to the support parts 115 and 117 (fixing step). The container connection part 111 may be fixed to the support parts 115 and 117 through a method such as laser bonding or ultrasonic bonding. By including the fixing step, it is possible to efficiently manufacture various types of drug mixing devices 100 by separately manufacturing the container connection part 111 suitable for the shape or size of the second container 7 to be coupled to the drug mixing device 100 and commonly producing the remaining portions of the body 110 including the support parts 115 and 117.

FIGS. 2A to 2F are vertical cross-sectional views illustrating a drug mixing device 100, a syringe 200, the first container 6, and/or the second container 7 taken along line S1-S1' in FIG. 1 and sequentially showing an example of using the drug mixing kit 1 of FIG. 1. Hereinafter, an example of use will be described in order with reference to FIGS. 2A to 2F.

Referring to FIG. 2A, the user prepares the drug mixing kit 1. As an example, the user may purchase the drug mixing kit 1 including the drug mixing device 100 and the syringe 200 coupled to each other. Thus, the user may prepare the drug mixing kit 1 without a separate operation of coupling the drug mixing device 100 and the syringe 200. As another example, the user may prepare the drug mixing kit 1 by purchasing each of the drug mixing device 100 and the syringe 200, and coupling the syringe 200 to the drug mixing device 100. In the latter case, the user may couple the syringe 200 to the drug mixing device 100 after coupling the second container 7 to the drug mixing device 100 and coupling the first container 6 to the syringe 200.

The user disposes the first container 6 and the second container 7, each of which contains a medically necessary content, in the syringe 200 and the drug mixing device 100, respectively. The user inserts the first container 6, which contains a first content C1 in a liquid form, into the introduction space 230s in the syringe 200. The user couples the second container 7, which contains a second content C2 in a liquid or powder form, to the drug mixing device 100. Here, the other end Q2 of the drug flow path P communicates the interior of the second container 7.

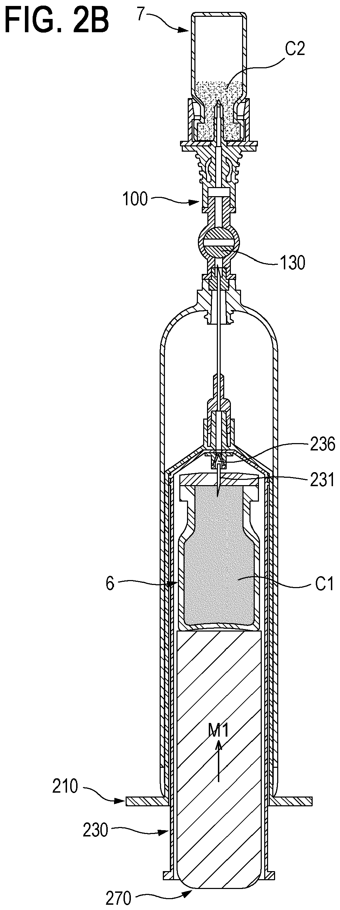

Referring to FIG. 2B, the user moves the rod 270 in the pressurizing direction M1 with respect to the plunger 230 so that the inner needle 235 is inserted into the first container 6. Here, the distal end in the first direction D1 of the inner flow path 230p communicates the interior of the first container 6.

Referring to FIG. 2C, in the state in which the valve closes the drug flow path P, when the user increases the volume of the accommodation space 200s by moving the plunger 230 with respect to the syringe housing 210 in the inflow motion direction M2, the first content C1 in the first container 6 flows into the accommodation space 200s through the inner flow path 230s *(see* arrow F1). Here, the pressure inside the first container 6 may be lowered, and even if the user releases the plunger 230 pulled in the inflow motion direction M2, the first content C1 is prevented from returning to the interior of the first container 6 by the backflow prevention part 236.

Referring to FIG. 2D, the user opens the drug flow path P by manipulating the valve 130. In the state in which the valve opens the drug flow path P, when the user decreases the volume of the accommodation space 200s by moving the plunger 230 with respect to the syringe housing 210 in the discharge motion direction M3, the first content C1 in the accommodation space 200s flows into the second container 7 through the drug flow path P (*see* arrow F2). Here, even if the user moves the plunger 230 in the discharge motion direction M3, the first content C1 is prevented from flowing into the first container 6 by the backflow prevention part 236. When the first content C1 flows into the second container 7, the user shakes the drug mixing kit 1 lightly to produce a mixture C3 of the first content C1 and the second content C2 in the second container 7.

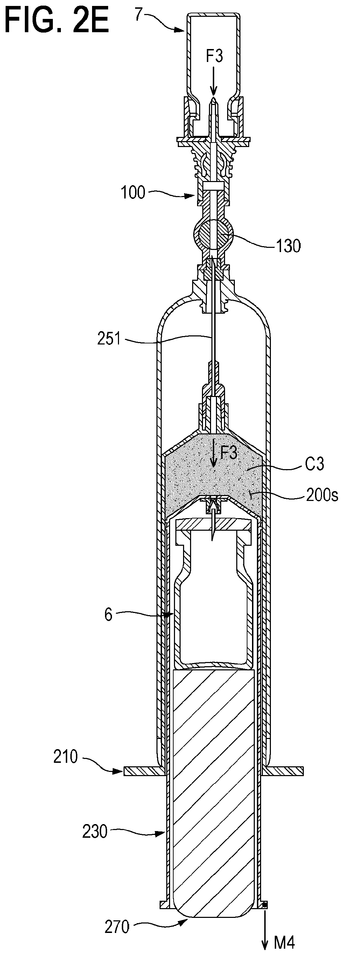

Referring to FIG. 2E, in the state in which the valve 130 opens the drug flow path P, when the user increases the volume of the accommodation space 200s by moving the plunger 230 with respect to the syringe housing 210 in the inflow motion direction M4, the mixture C3 in the second container 7 flows into the accommodation space 200s through the drug flow path P *(see* arrow F3).

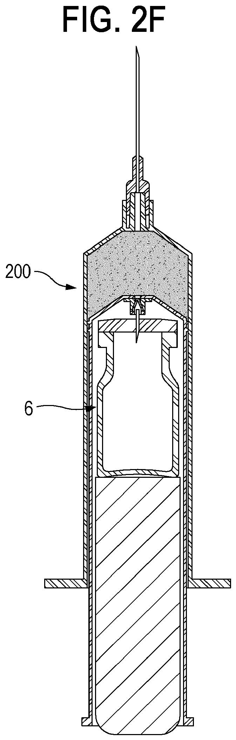

Referring to FIG. 2F, after separating the syringe 200 from the drug mixing device 100, the user may pierce the patient's skin with the syringe needle 250 to inject the mixture C3 as a drug to the patient.

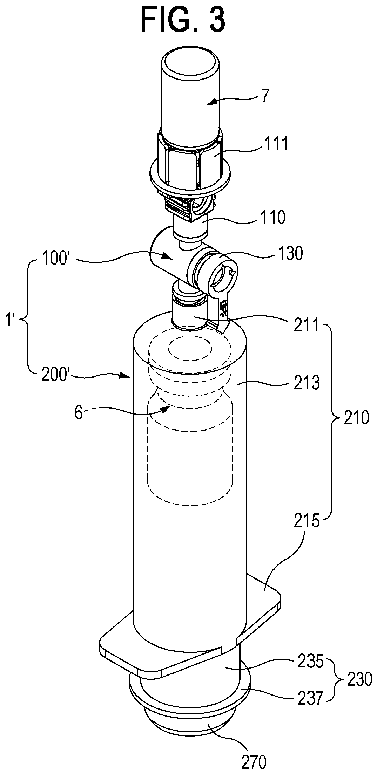

FIG. 3 is a perspective view illustrating the state in which a first container 6 and a second container 7 are coupled to a drug mixing kit 1' according to a second embodiment of the present disclosure. FIG. 4 is a vertical cross-sectional view illustrating the drug mixing kit 1' of FIG. 3.

Hereinafter, focusing on the differences from the first embodiment described above, the drug mixing kit 1', the drug mixing device 100', and the syringe 200' according to the second embodiment will be described with reference to FIGS. 3 and 4.

The drug mixing kit 1' may include a drug mixing device 100', which does not include the sealer and the supporter. The drug mixing kit 1' may include a syringe 200', which does not include the syringe needle.

The drug mixing device 100' and the syringe 200' are coupled to each other. The syringe connection part 118 of the drug mixing device 100' and the coupling part 211 of the syringe 200' are coupled to each other. In the second embodiment, the syringe connection part 118 and the coupling part 211 may be screw-fastened to each other. After filling the accommodation space 200s of the syringe 200' with the mixture C3 and then separating the syringe 200' from the drug mixing device 100', the user may couple a separate device for drug injection into the patient, such as a catheter or an injection needle, to the syringe 200'.

The technical idea of the present disclosure has been described heretofore with reference to some embodiments and examples shown in the accompanying drawings. However, it is to be understood that various substitutions, modifications and alterations may be made without departing from the technical idea and scope of the appended claims.

## Claims

1. A drug mixing kit (1) for mixing a first content (C1) in a liquid form in a first container (6) with a second content (C2) in a liquid or powder form in a second container (7), the drug mixing kit (1) comprising:
a syringe (200) forming therein an accommodation space (200s) configured to accommodate a liquid; and
a drug mixing device (100) detachably coupled to the syringe (200) and including a drug flow path (P) extending from one end configured to allow the liquid in the accommodation space (200s) to flow into the drug flow path (P) therethrough to the other end configured to allow the liquid or powder in the second container (7) to flow into the drug flow path (P) therethrough,
wherein the drug mixing device (100) includes
a body (110) including a container connection part (111) configured to be couplable with the second container (7) and a syringe connection part (118) configured to be connectable with the syringe (200); and
a valve (130) disposed in the drug flow path (P) and configured to open/close the liquid in the drug flow path (P),
**characterized in that** the syringe (200) includes:
a syringe housing (210) forming a space therein; and
a plunger (230) configured to change a volume of the accommodation space (200s) inside the syringe housing (210) by being inserted into and moving in the syringe housing (210), the plunger (230) forming therein an introduction space (230s) into which the first container (6) is introduced and an inner flow path (230p) through which the introduction space (230s) and the accommodation space (200s) communicate with each other,
wherein the plunger (230) includes
an inner needle (235) forming therein at least a portion of the inner flow path (230p) and protruding into the introduction space (230s); and
a backflow prevention part (236) disposed in the inner flow path (230p), and configured to prevent a flow of a liquid from a side of the accommodation space (200s) to a side of the introduction space (230s) while allowing a flow of the liquid from the side of the introduction space(230s) to the side of the accommodation space (200s), and
**characterized in that** the drug mixing device (100) further includes a sealer (180) disposed in a partial flow path (Pa) of the drug flow path (P) extending to a side of the one end with reference to a point (Q3) at which the valve (130) is disposed in the drug flow path (P), and
the syringe (200) further includes a syringe needle (250) configured to penetrate the sealer (180) and forming therein a flow path to guide a flow of the liquid between the drug flow path (P) and the accommodation space (200s).

2. The drug mixing kit(1) of claim 1, wherein the syringe (200) further includes a rod (270) configured to pressurize the first container (6) by being inserted into and moved in the introduction space (230s).

3. The drug mixing kit (1) of claim 1, wherein the plunger (230) is configured to open the introduction space (230s) in one direction, and the inner needle (235) protrudes in the one direction.

4. The drug mixing kit (1) of claim 3, wherein the plunger (230) is configured to be movable with respect to the syringe housing (210) in the one direction.

5. The drug mixing kit (1) of claim 1, wherein an outer flow path (200p) configured to guide a flow of the liquid between the accommodation space (200s) and an exterior of the syringe (200) is formed in the syringe (200).

6. The drug mixing kit (1) of claim 5, wherein the syringe needle (250) forms therein at least a portion of the outer flow path (200p) and protrudes to the exterior of the syringe housing (210).

7. The drug mixing kit (1) of claim 1, wherein the container connection part (111) includes a plurality of hooks (11a) configured to be engaged with the second container (7), and the body (110) includes a support part (115, 117) configured to support the container connection part (111).

8. The drug mixing kit (1) of claim 7, wherein the drug mixing device (100) further includes an outer needle (150) forming a side of the other end of the drug flow path (P) and protruding to be insertable into an interior of the second container (7), and
the plurality of hooks (111a) of the container connection part (111) are arranged to be spaced apart from each other in a circumferential direction around the outer needle (150).

9. The drug mixing kit(1) of claim 1, wherein the drug mixing device (100) further includes an outer needle (150) forming a side of the other end of the drug flow path (P) and protruding to be insertable into an interior of the second container (7).

10. The drug mixing kit (1) of claim 1, wherein the drug mixing device (100) further includes a supporter (190) coupled to the body (110) and configured to support the syringe (200).

## Patentansprüche

1. Arzneimittelmischkit (1) zum Mischen eines ersten Inhalts (C1) in einer flüssigen Form in einem ersten Behälter (6) mit einem zweiten Inhalt (C2) in einer flüssigen oder Pulverform in einem zweiten Behälter (7), wobei das Arzneimittelmischkit (1) umfasst:
eine Spritze (200), die in sich einen Aufnahmeraum (200s) bildet, der so konfiguriert ist, dass er eine Flüssigkeit aufnimmt; und
eine Arzneimittelmischvorrichtung (100), die lösbar mit der Spritze (200) gekoppelt ist und einen Arzneimittelströmungspfad (P) beinhaltet, der sich von einem Ende, das so konfiguriert ist, dass es die Flüssigkeit im Aufnahmeraum (200s) in den Arzneimittelströmungspfad (P) durch sie hindurch strömen lässt, zum anderen Ende erstreckt, das so konfiguriert ist, dass es die Flüssigkeit oder das Pulver im zweiten Behälter (7) in den Arzneimittelströmungspfad (P) durch sie hindurch strömen lässt,
wobei die Arzneimittelmischvorrichtung (100) beinhaltet
einen Körper (110), der einen Behälterverbindungsteil (111) beinhaltet, der so konfiguriert ist, dass er mit dem zweiten Behälter (7) gekoppelt werden kann, und einen Spritzenverbindungsteil (118), der so konfiguriert ist, dass er mit der Spritze (200) verbunden werden kann; und
ein Ventil (130), das im Arzneimittelströmungspfad (P) angeordnet und so konfiguriert ist, dass es die Flüssigkeit im Arzneimittelströmungspfad (P) öffnet/schließt,
**dadurch gekennzeichnet, dass** die Spritze (200) beinhaltet:
ein Spritzengehäuse (210), das in sich einen Raum bildet; und
einen Kolben (230), der so konfiguriert ist, dass er ein Volumen des Aufnahmeraums (200s) im Inneren des Spritzengehäuses (210) verändert, indem er in das Spritzengehäuse (210) eingebracht wird und sich darin bewegt, wobei der Kolben (230) in sich einen Einführraum (230s), in den der erste Behälter (6) eingeführt wird, und einen inneren Strömungspfad (230p) bildet, durch den der Einführraum (230s) und der Aufnahmeraum (200s) miteinander kommunizieren,
wobei der Kolben (230) beinhaltet
eine innere Nadel (235), die in sich mindestens einen Abschnitt des inneren Strömungspfades (230p) bildet und in den Einführraum (230s) vorspringt; und
einen Rückströmungsverhinderungsteil (236), der im inneren Strömungspfad (230p) angeordnet und so konfiguriert ist, dass er eine Strömung einer Flüssigkeit von einer Seite des Aufnahmeraums (200s) zu einer Seite des Einführraums (230s) verhindert, während er eine Strömung der Flüssigkeit von der Seite des Einführraums (230s) zur Seite des Aufnahmeraums (200s) zulässt, und
**dadurch gekennzeichnet, dass** die Arzneimittelmischvorrichtung (100) weiter eine Versiegelung (180) beinhaltet, die in einem Teilströmungspfad (Pa) des Arzneimittelströmungspfades (P) angeordnet ist, der sich zu einer Seite des einen Endes in Bezug auf einen Punkt (Q3), an dem das Ventil (130) im Arzneimittelströmungspfad (P) angeordnet ist, erstreckt, und
die Spritze (200) weiter eine Spritzennadel (250) beinhaltet, die so konfiguriert ist, dass sie die Versiegelung (180) durchdringt und in dieser einen Strömungspfad bildet, um eine Strömung der Flüssigkeit zwischen dem Arzneimittelströmungspfad (P) und dem Aufnahmeraum (200s) zu führen.

2. Arzneimittelmischkit (1) nach Anspruch 1, wobei die Spritze (200) weiter einen Stab (270) beinhaltet, der so konfiguriert ist, dass er den ersten Behälter (6) druckbeaufschlagt, indem er in den Einführraum (230s) eingebracht und darin bewegt wird.

3. Arzneimittelmischkit (1) nach Anspruch 1, wobei der Kolben (230) so konfiguriert ist, dass er den Einführraum (230s) in einer Richtung öffnet, und die innere Nadel (235) in der einen Richtung vorspringt.

4. Arzneimittelmischkit (1) nach Anspruch 3, wobei der Kolben (230) so konfiguriert ist, dass er in Bezug auf das Spritzengehäuse (210) in der einen Richtung beweglich ist.

5. Arzneimittelmischkit (1) nach Anspruch 1, wobei ein äußerer Strömungspfad (200p), der so konfiguriert ist, dass er eine Strömung der Flüssigkeit zwischen dem Aufnahmeraum (200s) und einer Außenseite der Spritze (200) führt, in der Spritze (200) gebildet ist.

6. Arzneimittelmischkit (1) nach Anspruch 5, wobei die Spritzennadel (250) in sich mindestens einen Abschnitt des äußeren Strömungspfades (200p) bildet und zur Außenseite des Spritzengehäuses (210) vorspringt.

7. Arzneimittelmischkit (1) nach Anspruch 1, wobei der Behälterverbindungsteil (111) eine Vielzahl von Haken (11a) beinhaltet, die so konfiguriert sind, dass sie mit dem zweiten Behälter (7) in Eingriff gebracht werden können, und der Körper (110) einen Trägerteil (115, 117) beinhaltet, der so konfiguriert ist, dass er den Behälterverbindungsteil (111) trägt.

8. Arzneimittelmischkit (1) nach Anspruch 7, wobei die Arzneimittelmischvorrichtung (100) weiter eine äußere Nadel (150) beinhaltet, die eine Seite des anderen Endes des Arzneimittelströmungspfades (P) bildet und so vorspringt, dass sie in einen Innenraum des zweiten Behälters (7) eingebracht werden kann, und
die Vielzahl von Haken (111a) des Behälterverbindungsteils (111) so angeordnet sind, dass sie in einer Umfangsrichtung um die äußere Nadel (150) herum voneinander beabstandet sind.

9. Arzneimittelmischkit (1) nach Anspruch 1, wobei die Arzneimittelmischvorrichtung (100) weiter eine äußere Nadel (150) beinhaltet, die eine Seite des anderen Endes des Arzneimittelströmungspfades (P) bildet und so vorspringt, dass sie in einen Innenraum des zweiten Behälters (7) eingebracht werden kann.

10. Arzneimittelmischkit (1) nach Anspruch 1, wobei die Arzneimittelmischvorrichtung (100) weiter einen Träger (190) beinhaltet, der mit dem Körper (110) gekoppelt und so konfiguriert ist, dass er die Spritze (200) trägt.

## Revendications

1. Kit de mélange de médicament (1) pour le mélange d'un premier contenu (C1) sous forme liquide dans un premier récipient (6) avec un second contenu (C2) sous forme liquide ou sous forme de poudre dans un second récipient (7), le kit de mélange de médicament (1) comprenant :
une seringue (200) formant en son sein un espace de réception (200s) configuré pour recevoir un liquide ; et
un dispositif de mélange de médicament (100) accouplé de manière amovible à la seringue (200) et incluant un trajet d'écoulement de médicament (P) s'étendant à partir d'une extrémité configurée pour permettre au liquide dans l'espace de réception (200s) de s'écouler dans le trajet d'écoulement de médicament (P) au travers vers l'autre extrémité configurée pour permettre au liquide ou à la poudre dans le second récipient (7) de s'écouler dans le trajet d'écoulement de médicament (P) au travers,
dans lequel le dispositif de mélange de médicament (100) inclut
un corps (110) incluant une partie de raccordement de récipient (111) configurée pour pouvoir être accouplée au second récipient (7) et une partie de raccordement de seringue (118) configurée pour pouvoir être raccordée à la seringue (200) ; et
une soupape (130) disposée dans le trajet d'écoulement de médicament (P) et configurée pour ouvrir/fermer le liquide dans le trajet d'écoulement de médicament (P),
**caractérisé en ce que** la seringue (200) inclut :
un logement de seringue (210) formant un espace en son sein ; et
un piston (230) configuré pour modifier un volume de l'espace de réception (200s) à l'intérieur du logement de seringue (210) en étant inséré et en se déplaçant dans le logement de seringue (210), le piston (230) formant en son sein un espace d'introduction (230s) dans lequel le premier récipient (6) est introduit et un trajet d'écoulement interne (230p) à travers lequel l'espace d'introduction (230s) et l'espace de réception (200s) communiquent l'un avec l'autre,
dans lequel le piston (230) inclut
une aiguille interne (235) formant en son sein au moins une partie du trajet d'écoulement interne (230p) et faisant saillie dans l'espace d'introduction (230s) ; et
une partie anti-reflux (236) disposée dans le trajet d'écoulement interne (230p), et configurée pour empêcher un écoulement d'un liquide à partir d'un côté de l'espace de réception (200s) vers un côté de l'espace d'introduction (230s) tout en permettant un écoulement du liquide à partir du côté de l'espace d'introduction (230s) vers le côté de l'espace de réception (200s), et
**caractérisé en ce que** le dispositif de mélange de médicament (100) inclut en outre un produit d'étanchéité (180) disposé dans un trajet d'écoulement partiel (Pa) du trajet d'écoulement de médicament (P) s'étendant vers un côté de l'extrémité par rapport à un point (Q3) au niveau duquel la soupape (130) est disposée dans le trajet d'écoulement de médicament (P), et la seringue (200) inclut en outre une aiguille de seringue (250) configurée pour pénétrer dans le produit d'étanchéité (180) et formant en son sein un trajet d'écoulement pour guider un écoulement du liquide entre le trajet d'écoulement de médicament (P) et l'espace de réception (200s).

2. Kit de mélange de médicament (1) selon la revendication 1, dans lequel la seringue (200) inclut en outre une tige (270) configurée pour mettre sous pression le premier récipient (6) en étant insérée et déplacée dans l'espace d'introduction (230s).

3. Kit de mélange de médicament (1) selon la revendication 1, dans lequel le piston (230) est configuré pour ouvrir l'espace d'introduction (230s) dans une direction, et l'aiguille interne (235) fait saillie dans la direction.

4. Kit de mélange de médicament (1) selon la revendication 3, dans lequel le piston (230) est configuré pour être mobile par rapport au logement de seringue (210) dans la direction.

5. Kit de mélange de médicament (1) selon la revendication 1, dans lequel un trajet d'écoulement externe (200p) configuré pour guider un écoulement du liquide entre l'espace de réception (200s) et un extérieur de la seringue (200) est formé dans la seringue (200).

6. Kit de mélange de médicament (1) selon la revendication 5, dans lequel l'aiguille de seringue (250) forme en son sein au moins une partie du trajet d'écoulement externe (200p) et fait saillie vers l'extérieur du logement de seringue (210).

7. Kit de mélange de médicament (1) selon la revendication 1, dans lequel la partie de raccordement de récipient (111) inclut une pluralité de crochets (11a) configurés pour être mis en prise avec le second récipient (7), et le corps (110) inclut une partie de support (115, 117) configurée pour supporter la partie de raccordement de récipient (111).

8. Kit de mélange de médicament (1) selon la revendication 7, dans lequel le dispositif de mélange de médicament (100) inclut en outre une aiguille externe (150) formant un côté de l'autre extrémité du trajet d'écoulement de médicament (P) et faisant saillie pour pouvoir être insérée dans un intérieur du second récipient (7), et
les crochets de la pluralité de crochets (111a) de la partie de raccordement de récipient (111) sont agencés pour être espacés les uns des autres dans une direction circonférentielle autour de l'aiguille externe (150).

9. Kit de mélange de médicament (1) selon la revendication 1, dans lequel le dispositif de mélange de médicament (100) inclut en outre une aiguille externe (150) formant un côté de l'autre extrémité du trajet d'écoulement de médicament (P) et faisant saillie pour pouvoir être insérée dans un intérieur du second récipient (7).

10. Kit de mélange de médicament (1) selon la revendication 1, dans lequel le dispositif de mélange de médicament (100) inclut en outre un dispositif de support (190) accouplé au corps (110) et configuré pour supporter la seringue (200).
